# EUROPEAN PATENT SPECIFICATION

(11) **EP 4 364 782 B1**
(45) Date of publication and mention of the grant of the patent: **19.11.2025**
(21) Application number: 23765134.4
(22) Date of filing: 02.06.2023
(51) Int. Cl.: A61M 37/00, A24F 40/42, A24B 15/167, A24B 15/24, A61K 31/465

(54) **ELECTRONIC DEVICE FOR DISCHARGING EMULSION**
ELEKTRONISCHE VORRICHTUNG ZUR EMULSIONSENTLADUNG
DISPOSITIF ÉLECTRONIQUE DE DÉCHARGE D'ÉMULSION

(30) Priority: 20.09.2022 KR 20220118816
(43) Date of publication of application: 08.05.2024
(73) Proprietor: KT&G Corporation, Daedeok-gu Daejeon 34337 (KR)
(72) Inventor: JEONG, Minseok, Daejeon 34128 (KR); KANG, Won Hyuk, Daejeon 34128 (KR); KIM, Jae Hyun, Daejeon 34128 (KR); CHUNG, Tae Young, Daejeon 34128 (KR)
(74) Representative: Ter Meer Steinmeister & Partner
(86) International application number: PCT/KR2023/007588
(87) International publication number: WO 2024/063248

(56) References cited:
- WO-A1-2021/207686
- CN-A- 113 368 385
- CN-A- 114 588 526
- JP-A- 2007 529 502
- JP-A- 2009 545 368
- JP-A- H 119 701
- US-A1- 2011 264 048
- US-A1- 2011 295 230
- US-A1- 2022 193 385

## Description

### Technical Field

The following embodiments relate to technology for releasing emulsion through an electronic device, and more particularly, to an electronic device attached to a skin of a user.

### Background Art

These days, there is a gradual rise in the demand for electronic cigarettes. The rising demand for electronic cigarettes has accelerated the continued development of electronic cigarette-related functions. The electronic cigarette-related functions may include, in particular, functions according to types and characteristics of electronic cigarettes. US 2011/295230 A1 relates to a micro-needle device comprising an active substance layer having a plurality of active substance chambers formed therein, a drive substance layer having a plurality of drive substance chambers formed therein and a needle support layer having a plurality of micro-needles extending therefrom. An activation layer comprising a plurality of heating elements for heating an expandable drive substance located in the drive substance chambers for in turn urging the second membrane into the active substance chamber for pressurising and urging an active substance therefrom through the corresponding micro-needle. A plurality of membrane accommodating recesses extend into the needle support layer for accommodating the first membrane as the active substance in the corresponding active substance chamber is pressurised. A puncturing member with a piercing point extends into each membrane accommodating recess. A barrier grating extends across each active substance chamber for preventing contact between the second membrane and the corresponding puncturing member. US 2011/264048 A1 relates to a micro-needle delivery device comprising an active substance accommodating layer having active substance accommodating chambers therein, a drive substance accommodating layer having drive substance accommodating chambers therein aligned with the active substance accommodating chambers, an activating layer having heating elements aligned with the drive substance accommodating chambers for heating a drive substance therein, and a micro-needle element having a needle support layer and a plurality of micro-needles extending therefrom. A burstable first membrane is located between and sealably secured to the micro-needle element and to the active substance accommodating layer for sealably closing the active substance accommodating chambers at one end. A second stretchable membrane is located between and sealably secured to the drive substance accommodating layer and to the active substance accommodating layer sealably closes the active substance accommodating chambers at the other end, and the drive substance accommodating chambers. A third membrane sealably closes the drive substance accommodating chambers. CN 114 588 526 A relates to the technical field of medical instruments, and provides a double-frequency ultrasonic microneedle array device which comprises a microneedle module, an acoustophoresis module and a connecting module. Array microneedles are arranged at the front end of the microneedle module, a connecting pipe connected with an injector is arranged on the side face of the microneedle module, and a cavity communicated with the array microneedles is formed in the microneedle module; the acoustophoresis module comprises a first vibrator and a second vibrator, and the connecting module is connected with the microneedle module, the first vibrator and the second vibrator. The diversity of vibrator structures can be achieved through the first vibrator and the second vibrator, in this way, the vibration mode can be changed by changing the phases of the first vibrator and the second vibrator, permeation of a medicament in the transverse direction and the depth direction of the skin can be accelerated by combining the array microneedles, the piercing force and tissue deformation are reduced, and the permeation effect is guaranteed. CN 113 368 385 A relates to a conveying system for delivering biological macromolecular drugs into a brain through a lymphatic pathway. The conveying system for delivering the biological macromolecular drugs into the brain through the lymphatic pathway comprises a low-pulse ultrasonic device, a conveying device, a microneedle device, a fixing device and a regulation and control device, wherein all the devices are modularly combined and can be detached and replaced, and the conveying device is used for conveying the macromolecular drugs to the microneedle device; the microneedle device is used for feeding the biological macromolecular drugs into the body of a patient through lymph nodes behind ears of the patient; ultrasonic waves emitted by the low-pulse ultrasonic device are used for stimulating and promoting the biological macromolecular drugs to permeate into the lymphatic pathway; the fixing device is used for fixing the conveying system to the lymph node part behind the ears of the patient; the regulation and control device is used for controlling the frequency and intensity of the ultrasonic waves and the ultrasonic duration. According to the conveying system for delivering the biological macromolecular drugs into the brain through the lymphatic pathway, a biological macromolecular drug W/O emulsion is delivered through the lymph pathway, so that the synergistic and complementary effects among low-pulse ultrasound, a hollow microneedle, the lymph brain-entering pathway and an efficient preparation are exerted, and the safe and efficient brain-entering effect of the biological macromolecular drugs is realized.

### Disclosure of the Invention

### Technical Solutions

According to the present invention, an electronic device for releasing emulsion through a microneedle includes a cartridge accommodation portion configured to accommodate a cartridge containing the emulsion therein, a crushing circuit including a crushing element configured to crush at least a portion of the cartridge disposed in the cartridge accommodation portion, a microneedle configured to release the emulsion to an outside of the electronic device, wherein the emulsion is exposed as at least the portion of the cartridge is crushed, a processor configured to generate a crushing signal, and a transmission circuit configured to transmit the crushing signal to the crushing circuit, wherein the crushing circuit may further include an ultrasonic vibrator, and the processor may be configured to generate a vibration signal for controlling the ultrasonic vibrator.

The crushing element is formed in the substrate of the crushing circuit to penetrate the substrate, and the crushing element may form a bump on a second surface of the substrate while forming a closed circuit with a first transmission element and a second transmission element of the transmission circuit on a first surface of the substrate.

A material of the crushing element may include a material that generates heat by the crushing signal, and at least the portion of the cartridge may be crushed by heat generated by the crushing element.

The material of the crushing element may be a metal having a specific resistance.

At least the portion of the cartridge crushed by the heat may be a thin-film cell.

The crushing circuit may further include an elastic element disposed between a bottom surface of a substrate of the crushing circuit and the cartridge.

The crushing circuit may further include an emulsion release element attached to the elastic element, and the elastic element and the emulsion release element may be configured to move the emulsion in the direction of the microneedle when at least the portion of the cartridge is crushed by the crushing element.

The electronic device may further include an indicator indicating an amount of emulsion to be released.

The electronic device may further include a button configured to receive, from a user, an amount of emulsion to be released.

The processor may be configured to, when the cartridge includes a plurality of blocks, each containing emulsion, generate the crushing signal to crush a first block of the plurality of blocks.

The crushing circuit may include a plurality of crushing elements, and the plurality of crushing elements may respectively correspond to the plurality of blocks.

The ultrasonic vibrator may include one or more ultrasonic vibrators, and the processor may be configured to generate a first vibration signal for controlling a first ultrasonic vibrator corresponding to the first block among the one or more ultrasonic vibrators.

According to an embodiment outside of the subject-matter of the claims, an electronic device for releasing emulsion through a microneedle may include a cartridge accommodation portion configured to accommodate a cartridge containing the emulsion therein, a crushing circuit including a crushing element configured to crush at least a portion of the cartridge disposed in the cartridge accommodation portion, a microneedle configured to release the emulsion to an outside of the electronic device, wherein the emulsion is exposed as at least the portion of the cartridge is crushed, a processor configured to generate a crushing signal, a transmission circuit configured to transmit the crushing signal to the crushing circuit, and an ultrasound transmission element configured to transmit ultrasound or ultrasonic vibration applied to one side of the electronic device to another side of the electronic device.

### Brief Description of Drawings

FIG. 1 illustrates a schematic diagram of an emulsion release system according to an example.
FIG. 2 illustrates a block diagram of an electronic device according to an embodiment.
FIG. 3A illustrates a connection relationship between a transmission circuit and a crushing circuit according to an embodiment.
FIG. 3B illustrates a connection relationship among a crushing circuit, a cartridge accommodation portion, and a microneedle portion according to an example.
FIG. 4 illustrates a connection relationship between a crushing circuit including an elastic element and a cartridge in a cartridge accommodation portion according to an example.
FIG. 5 illustrates a connection relationship between a cartridge accommodation portion and a microneedle portion according to an example.
FIG. 6 illustrates a crushing circuit including an infrared (IR) lamp, according to example.
FIG. 7 illustrates a cross-sectional view of an electronic device capable of transmitting external IR light in the direction of a skin of a user according to an example.
FIG. 8 illustrates a crushing circuit including an ultrasonic vibrator, according to an example.
FIG. 9 illustrates a cross-sectional view of an electronic device capable of transmitting external ultrasound in the direction of a skin of a user according to an example.
FIG. 10 is a flowchart of a method (not claimed) of releasing emulsion, according to an embodiment.
FIG. 11 is a flowchart of a method (not claimed) of generating a crushing signal based on a user input, according to an example.
FIG. 12 is a flowchart of a method (not claimed) of controlling an IR lamp, according to an example.
FIG. 13 is a flowchart of a method (not claimed) of controlling an ultrasonic vibrator, according to an example.

### Best Mode for Carrying Out the Invention

The following detailed structural or functional description is provided as an example only and various alterations and modifications may be made to embodiments. Accordingly, the embodiments are not to be construed as limited to the disclosure and should be understood to include all changes, equivalents, or replacements within the idea and the technical scope of the disclosure.

Although terms, such as first, second, and the like are used to describe various components, the components are not limited to the terms. These terms should be used only to distinguish one component from another component. For example, a first component may be referred to as a second component, and similarly the second component may also be referred to as the first component.

It should be noted that, if one component is described as being "connected," "coupled," or "joined" to another component, a third component may be "connected," "coupled," and "joined" between the first and second components, although the first component may be directly connected, coupled, or joined to the second component.

The singular forms "a," "an," and "the" are intended to include the plural forms as well, unless the context clearly indicates otherwise. It will be further understood that the terms "comprises/comprising" and/or "includes/including," when used herein, specify the presence of stated features, integers, steps, operations, elements, components, or combinations thereof, but do not preclude the presence or addition of one or more other features, integers, steps, operations, elements, components and/or combinations thereof.

Unless otherwise defined, all terms, including technical and scientific terms, used herein have the same meaning as commonly understood by one of ordinary skill in the art to which the present disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, embodiments are described in detail with reference to the accompanying drawings. When describing the embodiments with reference to the accompanying drawings, like reference numerals refer to like elements and a repeated description related thereto will be omitted.

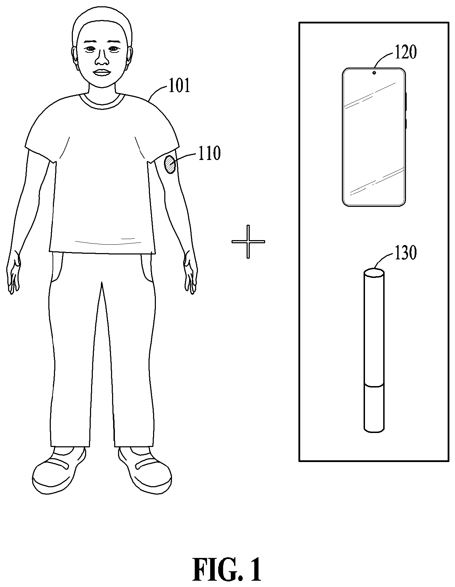

FIG. 1 illustrates a schematic diagram of an emulsion release system according to an example.

Referring to FIG. 1, the emulsion release system may include an electronic device 110 and an external device. For example, the external device may include a user terminal 120 (e.g., a smartphone) or a smokeless inhalation device 130 (e.g., an electronic cigarette or an inhaler).

According to an embodiment, the electronic device 110 may be attached to a part of the body of a user 101 and release emulsion (e.g., a liquid composition) onto or within the skin of the user 101. The emulsion released to the skin of the user 101 may be absorbed into the body of the user 101 through the skin. For example, the emulsion may include a medicinal component required for the user to treat a disease that the user 101 has. For example, the emulsion may include various vitamin components and mineral components. For example, the emulsion may include a component (e.g., nicotine) for the preferences of the user 101. For example, the acidity of the emulsion may be a value (e.g., between pH 7.0 and pH 8.0) that minimizes user irritation. When the pH of the emulsion is high, the emulsion may cause skin irritation due to its alkaline nature. When the pH of the emulsion is low, the emulsion may cause skin irritation due to its acidic nature. For example, the emulsion may be a liquid material containing water-soluble and lipid-soluble materials mixed with a surfactant in a predetermined ratio. The materials contained in the emulsion may be mixed to be evenly dispersed in the form of nano-sized droplets. In the present disclosure, the acidity of the emulsion is exemplified to be between pH 7.0 and pH 8.0. However, the acidity of the emulsion is not limited to the disclosed embodiment and may be adjusted to higher or lower values, considering skin absorption rate.

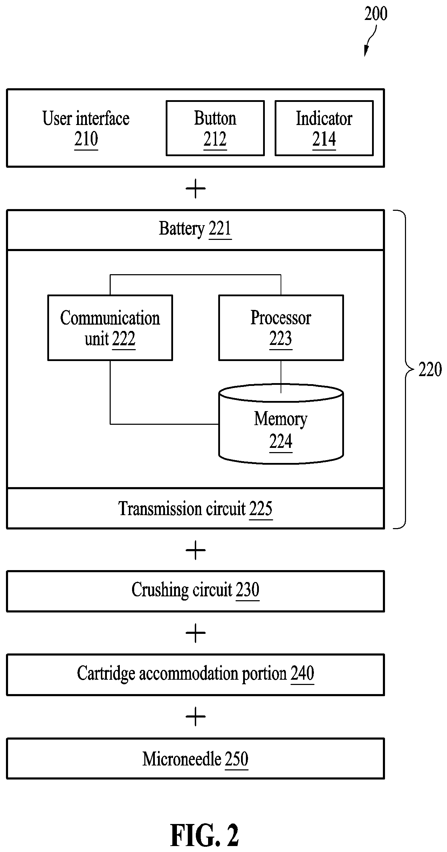

The electronic device 110 is described in detail below with reference to FIGS. 2 to 9.

According to an example, the electronic device 110 may be used independently as a standalone device. For example, the user 101 may directly input a control signal to the electronic device 110, and the electronic device 110 may control an operation of the electronic device 110 based on the control signal of the user 101.

According to an example, the electronic device 110 may establish a wireless communication connection with the user terminal 120 and receive the control signal from the user 101 through the user terminal 120.

According to an example, the electronic device 110 may establish a wireless communication connection with the smokeless inhalation device 130 and receive the control signal from the smokeless inhalation device 130. For example, when the user 101 uses the smokeless inhalation device 130, the smokeless inhalation device 130 may transmit a control signal corresponding to motion of the user to the electronic device 110. Upon receiving the control signal corresponding to the motion of the user, the electronic device 110 may release the emulsion to the user 101 based on the control signal.

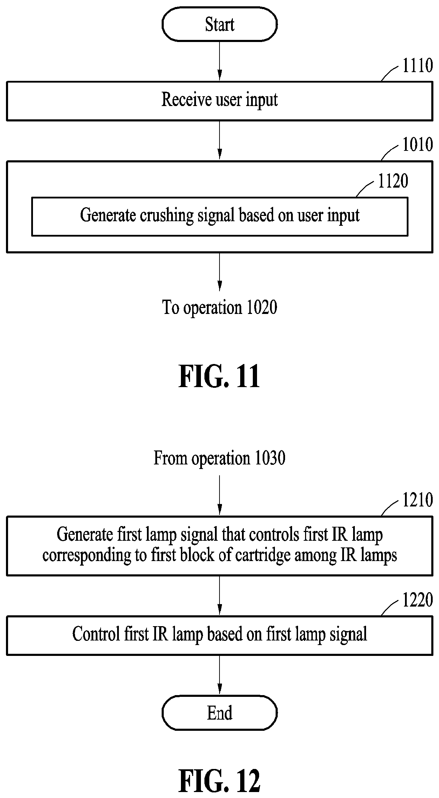

Various embodiments of the electronic device 110 receiving a user input are described in detail below with reference to FIG. 11.

FIG. 2 illustrates a block diagram of an electronic device according to an embodiment.

According to an embodiment, an electronic device 200 (e.g., the electronic device 110 of FIG. 1) may include a user interface 210, a controller 220, a crushing circuit 230, a cartridge accommodation portion 240, and a microneedle portion 250. The electronic device 200 may further include a fixing portion (e.g., a band, sticker, or layer) that provides a fixing force, allowing the electronic device 200 to be attached to a part of the body of a user. The fixing portion may be of a flexible type to enhance skin adherence. The material of the fixing portion may be a material (e.g., a porous polymer material) that does not cause skin irritation.

According to an embodiment, the user interface 210 may include a button 212 for physically receiving a user input and an indicator 214 (e.g., a light-emitting diode (LED)) for visually indicating a state of the electronic device 200. For example, the button 212 may receive, from the user, the amount of emulsion to be released. For example, the indicator 214 may indicate the amount of emulsion to be released. The user interface 210 may be disposed outside a housing of the electronic device 200.

According to an embodiment, the controller 220 may include a battery 221 that may supply power to the electronic device 200, a communication unit 222, a processor 223, a memory 224, and a transmission circuit 225. For example, the controller 220 may include a printed circuit board (PCB) that includes at least some of the battery 221, the communication unit 222, the processor 223, the memory 224, and the transmission circuit 225.

The communication unit 222 may be connected to the processor 223 and the memory 224 and transmit and receive data to and from the processor 223 and the memory 224. The communication unit 222 may transmit and receive data to and from other external devices (e.g., the user terminal 120 or the smokeless inhalation device 130 of FIG. 1) through short-range wireless communication. Hereinafter, transmitting and receiving "A" may refer to transmitting and receiving "information or data indicating A".

The communication unit 222 may be implemented as circuitry in the controller 220. For example, the communication unit 222 may include an internal bus and an external bus. In another example, the communication unit 222 may be an element that connects the controller 220 (or the electronic device 200) to an external device. The communication unit 222 may be an interface. The communication unit 222 may receive data from the external device and transmit the data to the processor 223 and the memory 224.

The processor 223 may process the data received by the communication unit 222 and data stored in the memory 224. A "processor" may be a hardware-implemented data processing device having a physically structured circuit to execute desired operations. The desired operations may include, for example, code or instructions included in a program. The hardware-implemented data processing device may include, for example, a microprocessor, a central processing unit (CPU), a processor core, a multi-core processor, a multiprocessor, an application-specific integrated circuit (ASIC), and a field-programmable gate array (FPGA).

The processor 223 may execute computer-readable code (e.g., software) stored in a memory (e.g., the memory 224) and instructions triggered by the processor 223.

The memory 224 may store data received by the communication unit 222 and data processed by the processor 223. For example, the memory 224 may store a program (or an application or software). The program to be stored may be a set of syntax that is coded and executable by the processor 223 to control the electronic device 200.

According to an aspect, the memory 224 may include at least one volatile memory, non-volatile memory, random-access memory (RAM), flash memory, a hard disk drive, and an optical disc drive.

The memory 224 may store an instruction set (e.g., software) for operating the controller 220. The instruction set for operating the controller 220 may be executed by the processor 223.

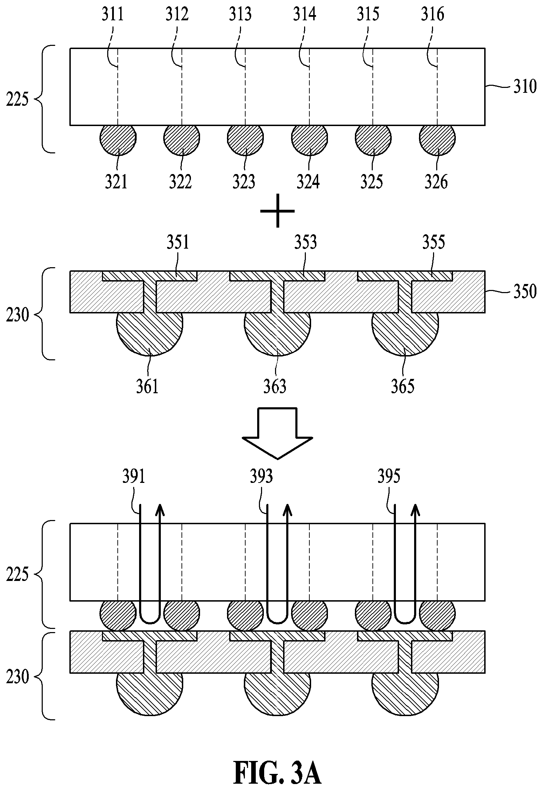

The transmission circuit 225 may transmit a signal (e.g., a crushing signal) generated by the processor 223 to the crushing circuit 230. The transmission circuit 225 is described in detail below with reference to FIG. 3A.

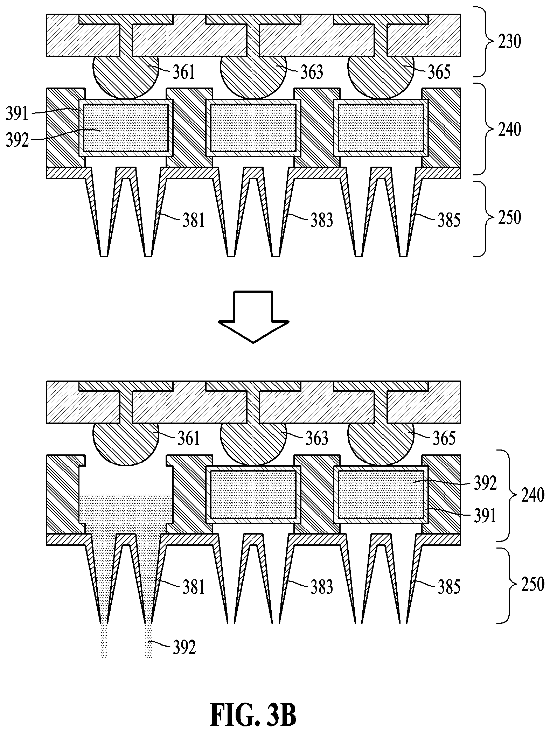

According to an embodiment, the crushing circuit 230 may crush at least a portion of the cartridge in the cartridge accommodation portion 240 based on the crushing signal transmitted through the transmission circuit 255. For example, a crushing element of the crushing circuit 230 may generate heat by the crushing signal, and at least the portion of the cartridge may be crushed by the heat. An embodiment in which at least the portion of the cartridge is crushed by the crushing element is described in detail below with reference to FIG. 3B.

According to an embodiment, the cartridge accommodation portion 240 may be configured to accommodate the cartridge therein. For example, the cartridge accommodation portion 240 may be formed such that one side of the cartridge disposed in the cartridge accommodation portion 240 comes into contact with the crushing element of the crushing circuit 230, while the other side of the cartridge is disposed in the direction of the microneedle portion 250. For example, the cartridge may include one or more blocks, and each of the blocks of the cartridge may be separately inserted into the cartridge accommodation portion 240. The cartridge or a block of the cartridge may be formed based on a thin-film cell that includes emulsion therein. For example, the thin-film cell of the cartridge may be manufactured based on a material (e.g., a polymer composite material or bio-based material) that may be crushed by heat. The emulsion within one block of the cartridge may contain an amount of material equivalent to that of a single typical cigarette. The structure of the cartridge accommodation portion 240 is described in detail below with reference to FIG. 3B.

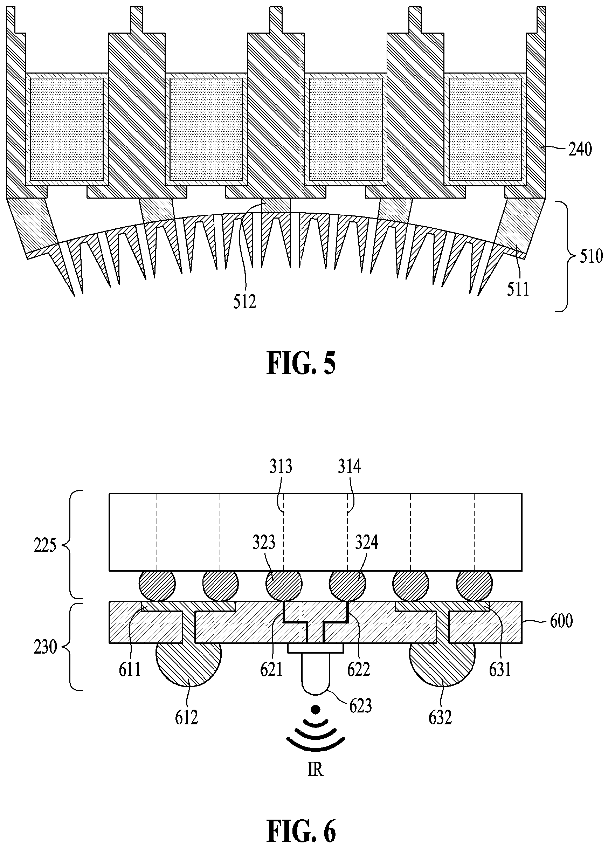

According to an embodiment, the microneedle portion 250 may include a plurality of microneedles. When a microneedle is attached to the skin of a user, the length of the microneedle may allow the microneedle to reach a dermis layer. For example, when the emulsion in the cartridge is exposed to the outside as the cartridge is crushed in the cartridge accommodation portion 240, the emulsion may be released into the dermis layer of the user through the microneedle portion 250. The emulsion released into the dermis layer may be absorbed into capillaries. The microneedle portion 250 may have a structure that may adhere well to the skin of the user. The structure of the microneedle portion 250 is described in detail below with reference to FIG. 5.

FIG. 3A illustrates a connection relationship between a transmission circuit and a crushing circuit according to an embodiment.

According to an embodiment, the transmission circuit 225 of FIG. 2 may include a substrate 310, wires 311, 312, 313, 314, 315, and 316 that pass through the substrate 310, and transmission elements 321, 322, 323, 324, 325, and 326 connected to the wires 311, 312, 313, 314, 315, and 316, respectively. For example, the transmission elements 321, 322, 323, 324, 325, and 326 may be in the form of a ball grid array (BGA). For example, the processor 223 may be positioned on the substrate 310, and pins of the processor 223 may be connected to the wires 311, 312, 313, 314, 315, and 316.

According to an embodiment, the crushing circuit 230 of FIG. 2 may include a substrate 350 and crushing elements formed to pass through the substrate 350. The crushing elements may include a plate and a bump. For example, a first crushing element may include a first plate 351 and a first bump 361, a second crushing element may include a second plate 353 and a second bump 363, and a third crushing element may include a third plate 355 and a third bump 365. A plate may be formed on a first surface of the substrate 350, and a bump electrically connected to the plate may be formed on a second surface of the substrate 350.

According to an embodiment, when the transmission circuit 225 is physically coupled to the crushing circuit 230, the first plate 351 of the first crushing element may be electrically connected to the first transmission element 321 and the second transmission element 322 of the transmission circuit 225. For example, the first plate 351 of the first crushing element may form a first closed circuit 391 with the first transmission element 321 and the second transmission element 322 of the transmission circuit 225 on the first surface of the substrate 350. For example, the second plate 353 of the second crushing element may form a second closed circuit 393 with the third transmission element 323 and the fourth transmission element 324 of the transmission circuit 225 on the first surface of the substrate 350. For example, the third plate 355 of the third crushing element may form a third closed circuit 395 with the fifth transmission element 325 and the sixth transmission element 326 of the transmission circuit 225 on the first surface of the substrate 350.

According to an embodiment, the material of the first crushing element may include a material that generates heat by a signal provided to a closed circuit. When power is supplied through the first closed circuit 391, the first crushing element may act as a resistance, resulting in the generation of heat in the first crushing element. For example, the material of the first crushing element may be a metal (e.g., copper) having a specific resistance.

FIG. 3B illustrates a connection relationship among a crushing circuit, a cartridge accommodation portion, and a microneedle portion according to an example.

According to an embodiment, a cartridge including a plurality of blocks may be inserted into the cartridge accommodation portion 240. For example, a first block 391 of the plurality of blocks may include a thin-film cell including emulsion 392 therein. Components of an emulsion included in each of the plurality of blocks may all be the same, or some components may be different.

According to an embodiment, while the cartridge is inserted into the cartridge accommodation portion 240, the crushing circuit 230 may be disposed on the cartridge accommodation portion 240. For example, the crushing circuit 230 may be coupled to the cartridge accommodation portion 240 such that the bumps 361, 363, and 365 of the crushing circuit 230 and thin-film cells of the cartridge contact each other. A plurality of crushing elements (e.g., the bumps 361, 363, and 365) may respectively correspond to the plurality of blocks of the cartridge in position.

According to an embodiment, the cartridge accommodation portion 240 may be coupled to the microneedle portion 250. For example, the cartridge accommodation portion 240 may be coupled to the microneedle portion 250 such that the emulsion may be released to the outside through a microneedle when the emulsion is exposed in the cartridge accommodation portion 240. For example, the microneedle portion 250 may include a plurality of microneedles 381, 383, and 385.

According to an embodiment, power may be supplied to the first crushing element or the first bump 361 through the first closed circuit 391 described above with reference to FIG. 3A. In this case, the first bump 361 may generate heat, and the generated heat may heat the first block 391 of the cartridge. When the first block 391 is exposed to heat beyond its heat resistance limit, the thin-film cell of the first block 391 may be crushed. When the thin-film cell of the first block 391 is crushed, the emulsion 392 in the thin-film cell may be released to the outside through the first microneedle 381 corresponding to the first block 391.

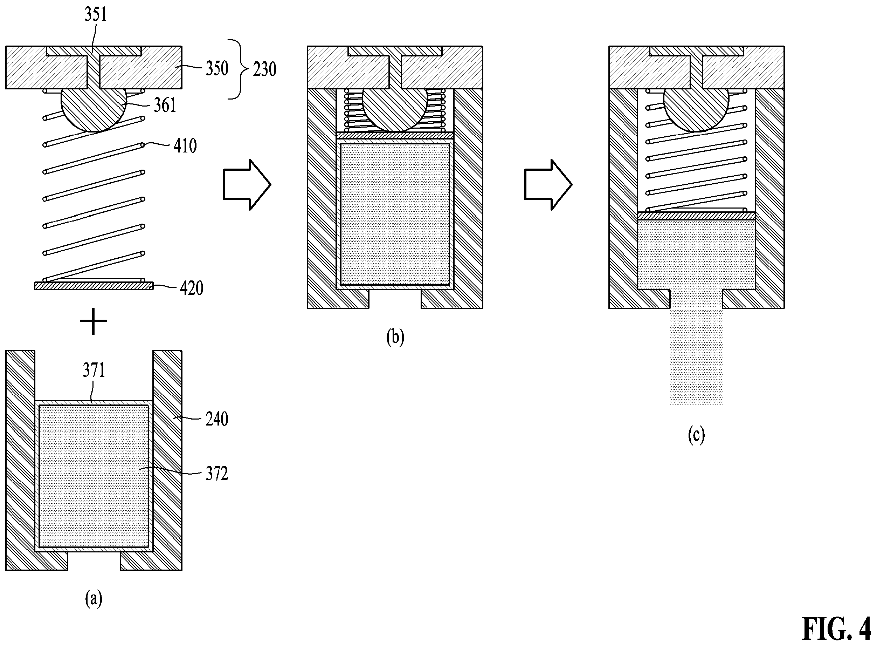

FIG. 4 illustrates a connection relationship between a crushing circuit including an elastic element and a cartridge in a cartridge accommodation portion according to an example.

According to an embodiment, the crushing circuit 230, described above with reference to FIGS. 2 and 3, may further include an elastic element (e.g., a first elastic element 410) and an emulsion release element (e.g., a first emulsion release element 420) in addition to a plate (e.g., the first plate 351) and a bump (e.g., the first bump 361). The elastic element may be disposed between one side (e.g., the bottom surface) of the substrate 350 of the crushing circuit 230 and a cartridge (e.g., the first block 371). For example, the elastic element may be a spring. The emulsion release element may be attached to the elastic element. For example, the material of the emulsion release element may include a material with high thermal conductivity.

According to an embodiment, in the course of coupling the crushing circuit 230 to the cartridge accommodation portion 240, the first elastic element 410 and the first emulsion release element 420 may be compressed by the first block 371 in the cartridge accommodation portion 240 in the direction of the first bump 361. For example, when the crushing circuit 230 is completely coupled to the cartridge accommodation portion 240, the first bump 361 and the first emulsion release element 420 contact each other, and the first emulsion release element 420 and the upper end of the first block 371 may contact each other.

According to an embodiment, when the first bump 361 generates heat, the generated heat may heat the first emulsion release element 420. The first block 371 may be crushed by the first emulsion release element 420 that is heated, and accordingly, the emulsion 372 in the first block 371 may be released to the outside through a microneedle. As the first block 371 is crushed, the first elastic element 410 may push the first emulsion release element 420 outward, and the emulsion 372 in the cartridge accommodation portion 240 may be released to the outside due to the movement of the first emulsion release element 420. In other words, when at least a portion (e.g., the first block 371) of the cartridge is crushed by the first crushing element (e.g., the first bump 361), the first elastic element 410 and the first emulsion release element 420 may move the emulsion 372 in the direction of the microneedle.

According to an embodiment, when the first emulsion release element 420 fills an end surface of the cartridge accommodation portion 240, leakage may be prevented since the emulsion 372 does not flow in the direction of the first bump 361. Furthermore, regardless of the placement or orientation of the electronic device 200, the emulsion 372 may be pushed in the direction of the microneedle.

FIG. 5 illustrates a connection relationship between a cartridge accommodation portion and a microneedle portion according to an example.

According to an embodiment, a microneedle portion 510 (e.g., the microneedle portion 250 of FIG. 2) may include a plurality of connecting units disposed between the cartridge accommodation portion 240 and a plurality of microneedles. For example, the length (or height) of each of the plurality of connecting units may vary depending on its placement location. For example, an outermost connecting unit 511 may be the longest among the rest of the connecting units, and a central connecting unit 512 may be the shortest. Due to the structure described above, the microneedle portion 510 may have an inwardly concave shape. When the microneedle portion 510 has an inwardly concave shape, the adhesion to the skin of a user may be enhanced. For example, the plurality of connecting units and the plurality of microneedles of the microneedle portion 510, which may be made from elastic materials, may adhere to the skin of the user.

FIG. 6 illustrates a crushing circuit including an infrared (IR) lamp, according to example.

According to an embodiment, the crushing circuit 230 described above with reference to FIG. 2 may include a substrate 600 and crushing elements formed to pass through the substrate 600. The crushing elements may include a plate and a bump. For example, a first crushing element may include a first plate 611 and a first bump 612 and a second crushing element may include a second plate 631 and a second bump 632. The plate may be formed on a first surface of the substrate 600, and the bump electrically connected to the plate may be formed on a second surface of the substrate 600.

According to an embodiment, the crushing circuit 230 may further include a first electrode 621, a second electrode 622, and an IR lamp 623 that emits IR light. The IR lamp 623 may be connected to the first electrode 621 and the second electrode 622. For example, the first electrode 621 and the second electrode 622 may be electrically and respectively connected to the third transmission element 323 and the fourth transmission element 324 of the transmission circuit 225 described above with reference to FIG. 3A. For example. the processor 223 may supply power to a closed circuit formed through the wires 313 and 314, the third transmission element 323, the fourth transmission element 324, the first electrode 621, the second electrode 622, and the IR lamp 623. When power is supplied to the closed circuit, the IR lamp 623 may emit IR light. More particularly, the emitted IR light may be near-IR (NIR). When NIR is irradiated to the skin of a user, NIR may reach a dermis layer of the skin.

According to an embodiment, when NIR is irradiated to the skin of the user, the absorption rate or absorption speed of the emulsion released to the skin may increase. For example, NIR may generate heat on the skin, and the generated heat may increase the absorption rate or absorption speed of the emulsion. For example, the heat generated by NIR may destroy nano-capsules in the emulsion in the dermis layer, allowing substances in the nano-capsules to be absorbed into capillaries.

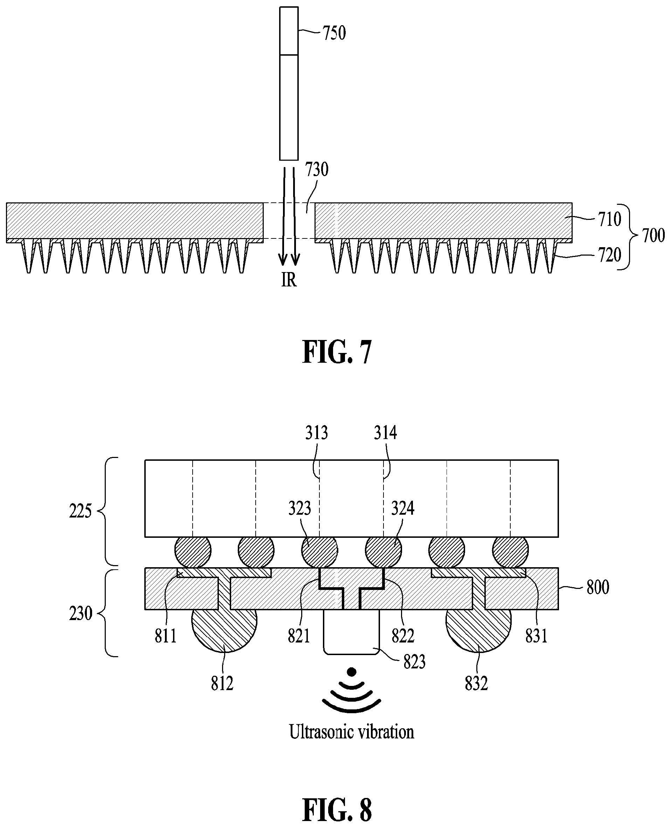

FIG. 7 illustrates a cross-sectional view of an electronic device capable of transmitting external IR light in the direction of the skin of a user, according to an example.

According to an embodiment, an electronic device 700 (e.g., the electronic device 100 of FIG. 1 or the electronic device 200 of FIG. 2) may include a body portion 710 and a microneedle portion 720. For example, the body portion 710 may include the user interface 210, the controller 220, the crushing circuit 230, and the cartridge accommodation portion 240.

According to an embodiment, the electronic device 700 may include a light transmission element 730 through which IR light may pass from one side (e.g., the upper surface) to the other side (e.g., the bottom surface) of the electronic device 700. For example, the shape of the light transmission element 730 may be a hole that penetrates the body portion 710 and the microneedle portion 720. For example, the light transmission element 730 may be an element made of a transparent material formed in the body portion 710 and the microneedle portion 720.

According to an embodiment, a user may irradiate IR light emitted by an IR emission device 750 onto one side of the electronic device 700. For example, the IR emission device 750 may be an electronic cigarette or an inhaler. IR light emitted by the IR emission device 750 may reach the other side of the electronic device 700 through the light transmission element 730 of the electronic device 700. In a state in which the electronic device 700 is attached to the skin of a user, when the user irradiates IR light onto the upper surface of the electronic device 700 using the IR emission device 750, the emitted IR light may reach the bottom surface of the electronic device 700 and thus reach the skin of the user.

FIG. 8 illustrates a crushing circuit including an ultrasonic vibrator, according to an example.

According to an embodiment, the crushing circuit 230 described above with reference to FIG. 2 may include a substrate 800 and crushing elements formed to pass through the substrate 800. The crushing elements may include a plate and a bump. For example, a first crushing element may include a first plate 811 and a first bump 812 and a second crushing element may include a second plate 831 and a second bump 832. The plate may be formed on a first surface of the substrate 800, and the bump electrically connected to the plate may be formed on a second surface of the substrate 800.

According to an embodiment, the crushing circuit 230 may further include a first electrode 821, a second electrode 822, and an ultrasonic vibrator 823 emitting ultrasound or ultrasonic vibrations. The ultrasonic vibrator 823 may be connected to the first electrode 821 and the second electrode 822. For example, the first electrode 821 and the second electrode 822 may be electrically and respectively connected to the third transmission element 323 and the fourth transmission element 324 of the transmission circuit 225 described above with reference to FIG. 3A. For example, the processor 223 may supply power to a closed circuit formed through the wires 313 and 314, the third transmission element 323, the fourth transmission element 324, the first electrode 821, the second electrode 822, and the ultrasonic vibrator 823. When power is supplied to the closed circuit, the ultrasonic vibrator 823 may emit ultrasound or ultrasonic vibrations.

According to an embodiment, the sonic energy of ultrasound or ultrasonic vibrations emitted onto the skin of a user may be converted into heat, and the heat may vaporize moisture within cells. The vaporized moisture within the cells may exert pressure on cellular tissue, causing spaces between the cells to expand. The expanded spaces between the cells may facilitate the absorption of emulsion.

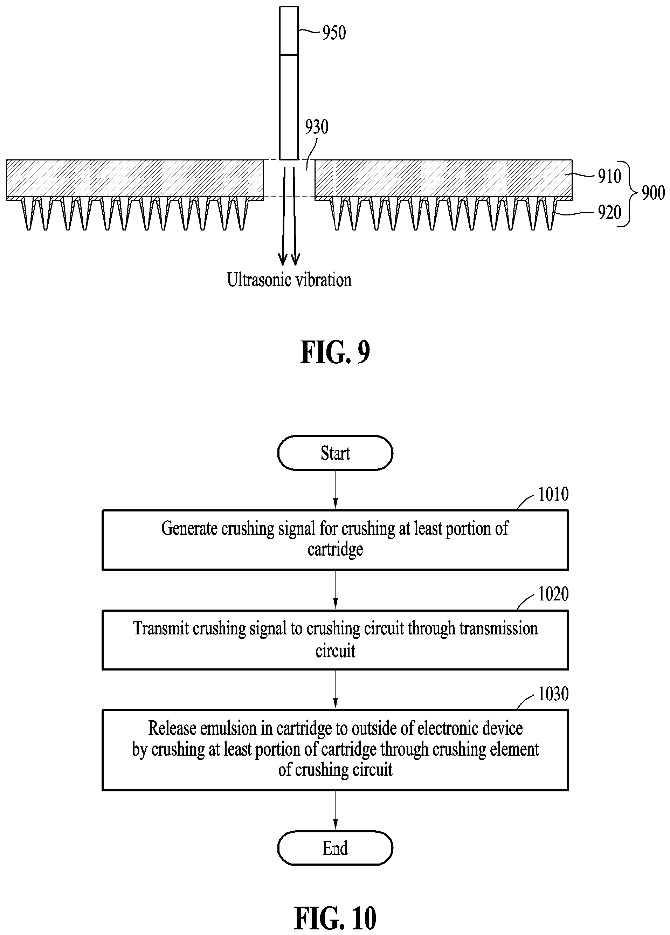

FIG. 9 illustrates a cross-sectional view of an electronic device capable of transmitting external ultrasound in the direction of the skin of a user, according to an example.

According to an embodiment, an electronic device 900 (e.g., the electronic device 100 of FIG. 1 or the electronic device 200 of FIG. 2) may include a body portion 910 and a microneedle portion 920. For example, the body portion 910 may include the user interface 210, the controller 220, the crushing circuit 230, and the cartridge accommodation portion 240.

According to an embodiment, the electronic device 900 may include an ultrasound transmission element 930 through which ultrasound or ultrasonic vibrations may pass from one side (e.g., the upper surface) to the other side (e.g., the bottom surface) of the electronic device 900. For example, the shape of the ultrasound transmission element 930 may be a hole that penetrates the body portion 910 and the microneedle portion 920. For example, the ultrasound transmission element 930 may be an element made of a transparent material formed in the body portion 910 and the microneedle portion 920.

According to an embodiment, a user may irradiate ultrasound emitted by an ultrasound emission device 950 onto one side of the electronic device 900. For example, the ultrasound emission device 950 may be an electronic cigarette or an inhaler. Ultrasound emitted by the ultrasound emission device 950 may reach the other side of the electronic device 900 through the ultrasound transmission element 930 of the electronic device 900. In a state in which the electronic device 900 is attached to the skin of a user, when the user irradiates ultrasound onto the upper surface of the electronic device 900 using the ultrasound emission device 950, the emitted ultrasound may reach the bottom surface of the electronic device 900 and thus reach the skin of the user.

FIG. 10 is a flowchart of a method of releasing emulsion, according to an embodiment.

Operations 1010 to 1030 may be performed by an electronic device (e.g., the electronic device 110 of FIG. 1 or the electronic device 200 of FIG. 2).

In operation 1010, the electronic device may generate a crushing signal for crushing at least a portion (e.g., the first block 391 of FIG. 3A) of a cartridge accommodated in the electronic device.

According to an embodiment, the electronic device may receive a user input from a user through a button (e.g., the button 212 of FIG. 2) of the electronic device and generate the crushing signal based on the user input. For example, the user input may include information (e.g., immediate or a timer) about the time at which the crushing signal is generated and information about the amount of emulsion to be released. For example, a single press of the button by a user may indicate a first emulsion amount, and two presses of the button by the user may indicate a second emulsion amount (e.g., twice the first emulsion amount).

For example, button A may be a button for the user to turn on the electronic device. For example, button B may be a button for the user to input the amount of emulsion (e.g., 0.1 milligrams (mg) when the button is pressed once, 0.3 mg when the button is pressed twice, and 0.5 mg when the button is pressed three times). For example, button C may be a button for the user to input an emulsion release command. According to an example, one button may perform one or more functions. The user may input different commands by pressing a button in different patterns.

According to an embodiment, information input by the user may be visually indicated through an indicator (e.g., the indicator 214 of FIG. 2). For example, the indicator may include a plurality of LEDs, and each LED may indicate a current state (e.g., a power state or emulsion release progress state) of the electronic device or a set amount of emulsion.

According to an embodiment, the electronic device may receive the user input from an external device connected to the electronic device through wireless communication. For example, the external device may include the user terminal 120 (e.g., a smartphone) or the smokeless inhalation device 130 (e.g., an electronic cigarette or an inhaler) of FIG. 1. A method of receiving the user input from the external device is described in detail below with reference to FIG. 11.

According to an embodiment, the electronic device may monitor a state of the user through a sensor of the electronic device disposed on the skin of the user and generate a crushing signal based on a monitoring result. For example, when the sensor of the electronic device is a blood glucose sensor, the electronic device may monitor blood glucose of the user using the blood glucose sensor. When the level of blood glucose of the user is greater than a preset value, the crushing signal of the cartridge may be generated such that emulsion containing insulin is supplied to the user.

According to an embodiment, the crushing signal may be generated such that a preset amount of emulsion is released at a preset time. For example, the time at which the crushing signal is generated by the user may be set in advance. For example, the time may be set in advance such that 0.2 mg of emulsion is released at 8 o'clock, 12 o'clock, and 7 o'clock. For example, when 0.1 mg of emulsion is accommodated in one block of the cartridge, the crushing signal may be generated such that two blocks are crushed at each target time.

According to an embodiment, the generation of the crushing signal may involve supplying power to a closed circuit (e.g., the first closed circuit 391, the second closed circuit 393, and/or the third closed circuit 395 of FIG. 3A) formed by a transmission circuit (e.g., the transmission circuit 225 of FIG. 2) and a crushing circuit (e.g., the crushing circuit 230 of FIG. 2), thereby allowing a current to flow through the closed circuit.

In operation 1020, the electronic device may transmit the generated crushing signal to the crushing circuit through the transmission circuit. For example, the crushing signal may be transmitted to the crushing circuit only through the first closed circuit 391 of a plurality of closed circuits. In this case, power may be supplied only to the first crushing element of the crushing circuit. When power is supplied to the first crushing element, a first bump (e.g., the first bump 361 of FIG. 3A) of the first crushing element may generate heat.

In operation 1030, the electronic device may release the emulsion in the cartridge to the outside by crushing at least one portion (e.g., the first block 391 of FIG. 3B) of the cartridge through a crushing element of the crushing circuit. For example, the emulsion in a block of the crushed cartridge may be released to the outside of the electronic device through a microneedle (e.g., the first microneedle 381 of FIG. 3B) disposed to correspond to the block. When the electronic device is attached to the skin of the user, the emulsion released to the outside of the electronic device may be absorbed into the capillaries of the user.

FIG. 11 is a flowchart of a method of generating a crushing signal based on a user input, according to an example.

According to an embodiment, operation 1110 may be performed before operation 1010 described above with reference to FIG. 10 is performed.

In operation 1110, an electronic device (e.g., the electronic device 110 of FIG. 1 or the electronic device 200 of FIG. 2) may receive a user input.

According to an embodiment, the electronic device may receive the user input through a user interface (e.g., the user interface 210 of FIG. 2) of the electronic device.

According to an embodiment, the electronic device may receive the user input through an external device (e.g., the user terminal 120 (e.g., a smartphone) or the smokeless inhalation device 130 (e.g., an electronic cigarette or an inhaler) of FIG. 1) through a communication unit (e.g., the communication unit 222 of FIG. 2). For example, the communication unit may establish a wireless communication channel with the external device and receive the user input from the external device through the wireless communication channel.

According to an embodiment, a user of an inhaler may set the amount of emulsion to be released through an input device such as a touch/button of the inhaler, and the inhaler may transmit the user input to the electronic device through the wireless communication channel. For example, when inhalation or a puff by the user is detected through the inhaler, the inhaler may transmit a preset user input to the electronic device through the wireless communication channel.

According to an embodiment, a user of a smartphone may input a user input to the electronic device through an application for controlling the electronic device installed on the smartphone. For example, the user may set a time at which emulsion is released, the amount of emulsion, and the like through the application of the smartphone. For example, a doctor associated with the user may transmit the user input to the electronic device through the user terminal such that the emulsion for disease treatment is supplied to the user at the right time and in the appropriate amount.

According to an embodiment, operation 1010 described above with reference to FIG. 10 may include operation 1120 below.

In operation 1120, the electronic device may generate a crushing signal based on a user input. For example, when the user input includes conditions for generating the crushing signal and the conditions are satisfied, the electronic device may generate the crushing signal.

FIG. 12 is a flowchart of a method of controlling an IR lamp, according to an example.

According to an embodiment, operations 1210 and 1220 may be further performed after operation 1030 described above with reference to FIG. 10 is performed. An electronic device (e.g., the electronic device 110 of FIG. 1 or the electronic device 200 of FIG. 2) may perform operations 1210 and 1220.

In operation 1210, the electronic device may generate a first lamp signal that controls a first IR lamp (e.g., the IR lamp 623 of FIG. 6) corresponding to a first block of a cartridge among one or more IR lamps. For example, the first block may be a block of a plurality of blocks of the cartridge that is crushed. When the electronic device includes a plurality of IR lamps, the first lamp signal that controls at least some of the IR lamps corresponding to the first block may be generated. When the electronic device includes one IR lamp, a lamp signal that controls an IR lamp may always be generated regardless of which block emulsion is being released from.

In operation 1220, the electronic device may control the first IR lamp based on the first lamp signal. For example, the first lamp signal may be a signal (e.g., power) supplied to a closed circuit formed through the wires 313 and 314, the third transmission element 323, the fourth transmission element 324, the first electrode 621, the second electrode 622, and the IR lamp 623 described above with reference to FIG. 6. The first IR lamp that receives the signal may emit IR light. For example, IR light may be emitted onto the skin of a user.

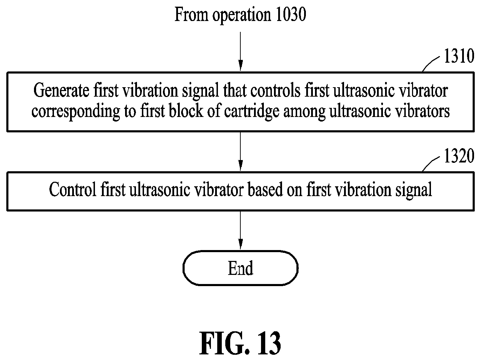

FIG. 13 is a flowchart of a method of controlling an ultrasonic vibrator, according to an example.

According to an embodiment, operations 1310 and 1320 may be further performed after operation 1030 described above with reference to FIG. 10 is performed. An electronic device (e.g., the electronic device 110 of FIG. 1 or the electronic device 200 of FIG. 2) may perform operations 1310 and 1320.

In operation 1310, the electronic device may generate a first vibration signal that controls a first ultrasonic vibrator (e.g., the ultrasonic vibrator 823 of FIG. 8) corresponding to a first block of a cartridge among one or more ultrasonic vibrators. For example, the first block may be a block of a plurality of blocks of the cartridge that is crushed. When the electronic device includes a plurality of ultrasonic vibrators, the first vibration signal that controls at least some of the ultrasonic vibrators corresponding to the first block may be generated. When the electronic device includes one ultrasonic vibrator, a vibration signal that controls the ultrasonic vibrator may be always generated regardless of which block emulsion is being released from.

In operation 1220, the electronic device may control the first ultrasonic vibrator based on the first vibration signal. For example, the first vibration signal may be a signal (e.g., power) supplied to a closed circuit formed through the wires 313 and 314, the third transmission element 323, the fourth transmission element 324, the first electrode 821, the second electrode 822, and the ultrasonic vibrator 823 described above with reference to FIG. 8. The first ultrasonic vibrator that receives the signal may release ultrasound or ultrasonic vibrations. For example, ultrasound or ultrasonic vibrations may be transmitted onto the skin of a user.

The methods according to the embodiments may be recorded in non-transitory computer-readable media including program instructions to implement various operations of the embodiments. The media may also include, alone or in combination with the program instructions, data files, data structures, and the like. The program instructions recorded on the media may be those specially designed and constructed for the purposes of embodiments, or they may be of the kind well-known and available to one of ordinary skill in the computer software arts. Examples of non-transitory computer-readable media include magnetic media such as hard disks, floppy disks, and magnetic tape; optical media such as CD-ROM discs or DVDs; magneto-optical media such as floptical disks; and hardware devices that are specially configured to store and perform program instructions, such as read-only memory (ROM), RAM, flash memory, and the like. Examples of program instructions include both machine code, such as produced by a compiler, and files containing higher-level code that may be executed by the computer using an interpreter. The above-described hardware devices may be configured to act as one or more software modules in order to perform the operations of the embodiments, or vice versa.

The software may include a computer program, a piece of code, an instruction, or one or more combinations thereof, to independently or collectively instruct or configure the processing device to operate as desired. Software and/or data may be embodied permanently or temporarily in any type of machine, component, physical or virtual equipment, computer storage medium or device, or in a propagated signal wave capable of providing instructions or data to or being interpreted by the processing device. The software may also be distributed over network-coupled computer systems so that the software may be stored and executed in a distributed fashion. The software and data may be stored by one or more non-transitory computer-readable recording mediums.

Although the embodiments have been described with reference to the limited drawings, one of ordinary skill in the art may apply various technical modifications and variations based thereon.

Therefore, other implementations, and other embodiments are also within the scope of the following claims.

## Claims

1. An electronic device (110) for releasing emulsion (372, 392) through a microneedle, the electronic device comprising:
a cartridge accommodation portion (240) configured to accommodate a cartridge containing the emulsion therein;
a crushing circuit (230) comprising a crushing element (361, 363, 365) configured to crush at least a portion of the cartridge disposed in the cartridge accommodation portion;
a microneedle (381, 383, 385) configured to release the emulsion to an outside of the electronic device, wherein the emulsion is exposed as at least the portion of the cartridge is crushed;
a processor (223) configured to generate a crushing signal; and
a transmission circuit (225) configured to transmit the crushing signal to the crushing circuit,
wherein the crushing circuit further comprises an ultrasonic vibrator (823), and
wherein the processor is configured to generate a vibration signal for controlling the ultrasonic vibrator.

2. The electronic device of claim 1, wherein
the crushing element is formed in a substrate (350, 800) of the crushing circuit to penetrate the substrate, and
the crushing element is configured to form a bump on a second surface of the substrate while forming a closed circuit with a first transmission element and a second transmission element of the transmission circuit on a first surface of the substrate.

3. The electronic device of claim 1, wherein
a material of the crushing element comprises a material that generates heat by the crushing signal, and
at least the portion of the cartridge is crushed by heat generated by the crushing element.

4. The electronic device of claim 3, wherein the material of the crushing element is a metal having a specific resistance.

5. The electronic device of claim 3, wherein at least the portion of the cartridge crushed by the heat is a thin-film cell.

6. The electronic device of claim 1, wherein the crushing circuit further comprises an elastic element (410) disposed between a bottom surface of a substrate of the crushing circuit and the cartridge.

7. The electronic device of claim 6, wherein
the crushing circuit further comprises an emulsion release element (420) attached to the elastic element, and
the elastic element and the emulsion release element are configured to move the emulsion in a direction of the microneedle when at least the portion of the cartridge is crushed by the crushing element.

8. The electronic device of claim 1, further comprising:
an indicator (214) indicating an amount of emulsion to be released.

9. The electronic device of claim 1, further comprising:
a button (212) configured to receive, from a user, an amount of emulsion to be released.

10. The electronic device of claim 1, wherein the processor is configured to, when the cartridge comprises a plurality of blocks, each containing emulsion, generate the crushing signal to crush a first block (371, 391) of the plurality of blocks.

11. The electronic device of claim 10, wherein
the crushing circuit comprises a plurality of crushing elements, and
the plurality of crushing elements respectively corresponds to the plurality of blocks.

12. The electronic device of claim 10, wherein
the ultrasonic vibrator comprises one or more ultrasonic vibrators, and
the processor is configured to generate a first vibration signal for controlling a first ultrasonic vibrator corresponding to the first block among the one or more ultrasonic vibrators.

## Patentansprüche

1. Elektronische Vorrichtung (110) zum Freigeben einer Emulsion (372, 392) durch eine Mikronadel, wobei die elektronische Vorrichtung umfasst:
einen Kassettenaufnahmeabschnitt (240), der konfiguriert ist, eine Kassette aufzunehmen, in der die Emulsion enthalten ist;
eine Zerkleinerungsschaltung (230), die ein Zerkleinerungselement (361, 363, 365) umfasst, das konfiguriert ist, zumindest einen Teil der Kassette, die in dem Kassettenaufnahmeabschnitt angeordnet ist, zu zerkleinern;
eine Mikronadel (381, 382, 385), die konfiguriert ist, die Emulsion zu einer Außenseite der elektronischen Vorrichtung freizugeben, wobei die Emulsion freigegeben wird, wenn zumindest der Teil der Kassette zerkleinert wird;
einen Prozessor (223), der konfiguriert ist, ein Zerkleinerungssignal zu erzeugen; und
eine Übertragungsschaltung (225), die konfiguriert ist, das Zerkleinerungssignal an die Zerkleinerungsschaltung zu übertragen,
wobei die Zerkleinerungsschaltung ferner einen Ultraschallschwingungserzeuger (823) umfasst und
wobei der Prozessor konfiguriert ist, ein Schwingungssignal zum Steuern des Ultraschallschwingungserzeugers zu erzeugen.

2. Elektronische Vorrichtung nach Anspruch 1, wobei
das Zerkleinerungselement in einem Substrat (350, 800) der Zerkleinerungsschaltung gebildet ist, um das Substrat zu durchdringen, und
das Zerkleinerungselement konfiguriert ist, einen Hügel auf einer zweiten Fläche des Substrats zu bilden, während es eine geschlossene Schaltung mit einem ersten Übertragungselement und einem zweiten Übertragungselement der Übertragungsschaltung auf einer ersten Fläche des Substrats bildet.

3. Elektronische Vorrichtung nach Anspruch 1, wobei
ein Material des Zerkleinerungselements ein Material umfasst, das durch das Zerkleinerungssignal Wärme erzeugt, und
zumindest der Teil der Kassette durch Wärme, die durch das Zerkleinerungselement erzeugt wird, zerkleinert wird.

4. Elektronische Vorrichtung nach Anspruch 3, wobei das Material des Zerkleinerungselements ein Metall mit einem spezifischen Widerstand ist.

5. Elektronische Vorrichtung nach Anspruch 3, wobei zumindest der Teil der Kassette, der durch die Wärme zerkleinert wird, eine Dünnschichtzelle ist.

6. Elektronische Vorrichtung nach Anspruch 1, wobei eine Zerkleinerungsschaltung ferner ein elastisches Element (410) umfasst, das zwischen einer unteren Fläche eines Substrats der Zerkleinerungsschaltung und der Kassette angeordnet ist.

7. Elektronische Vorrichtung nach Anspruch 6, wobei
die Zerkleinerungsschaltung ferner ein Emulsionsfreigabeelement (420) umfasst, das an dem elastischen Element angebracht ist, und
das elastische Element und das Emulsionsfreigabeelement konfiguriert sind, die Emulsion in Richtung der Mikronadel zu bewegen, wenn zumindest der Teil der Kassette durch das Zerkleinerungselement zerkleinert wird.

8. Elektronische Vorrichtung nach Anspruch 1, die ferner umfasst:
einen Indikator (214), der eine freizugebende Menge der Emulsion angibt.

9. Elektronische Vorrichtung nach Anspruch 1, die ferner umfasst:
einen Knopf (212), der konfiguriert ist, von einem Anwender eine freizugebende Menge der Emulsion zu empfangen.

10. Elektronische Vorrichtung nach Anspruch 1, wobei der Prozessor konfiguriert ist, dann, wenn die Kassette mehrere Blöcke umfasst, die jeweils eine Emulsion enthalten, das Zerkleinerungssignal zu erzeugen, um einen ersten Block (371, 391) der mehreren Blöcke zu zerkleinern.

11. Elektronische Vorrichtung nach Anspruch 10, wobei
die Zerkleinerungsschaltung mehrere Zerkleinerungselemente umfasst und
die mehreren Zerkleinerungselemente jeweils den mehreren Blöcken entsprechen.

12. Elektronische Vorrichtung nach Anspruch 10, wobei
der Ultraschallschwingungserzeuger einen oder mehrere Ultraschallschwingungserzeuger umfasst und
der Prozessor konfiguriert ist, ein erstes Schwingungssignal zum Steuern eines ersten Ultraschallschwingungserzeugers zu erzeugen, das dem ersten Block unter dem einen oder den mehreren Ultraschallschwingungserzeugern entspricht.

## Revendications

1. Dispositif électronique (110) pour libérer une émulsion (372, 392) à travers une micro-aiguille, le dispositif électronique comportant :
une partie de réception de cartouche (240) configurée pour recevoir une cartouche contenant l'émulsion dans celle-ci ;
un circuit d'écrasement (230) comportant un élément d'écrasement (361, 363, 365) configuré pour écraser au moins une partie de la cartouche disposée dans la partie de réception de cartouche ;
une micro-aiguille (381, 383, 385) configurée pour libérer l'émulsion vers un extérieur du dispositif électronique, dans lequel l'émulsion est exposée lorsqu'au moins la partie de la cartouche est écrasée ;
un processeur (223) configuré pour générer un signal d'écrasement ; et
un circuit de transmission (225) configuré pour transmettre le signal d'écrasement au circuit d'écrasement,
dans lequel le circuit d'écrasement comporte en outre un vibreur à ultrasons (823), et
dans lequel le processeur est configuré pour générer un signal de vibration pour commander le vibreur à ultrasons.

2. Dispositif électronique selon la revendication 1, dans lequel
l'élément d'écrasement est formé dans un substrat (350, 800) du circuit d'écrasement pour pénétrer dans le substrat, et
l'élément d'écrasement est configuré pour former une bosse sur une seconde surface du substrat tout en formant un circuit fermé avec un premier élément de transmission et un second élément de transmission du circuit de transmission sur une première surface du substrat.

3. Dispositif électronique selon la revendication 1, dans lequel
un matériau de l'élément d'écrasement comporte un matériau qui génère de la chaleur par le signal d'écrasement, et
au moins la partie de la cartouche est écrasée par la chaleur générée par l'élément d'écrasement.

4. Dispositif électronique selon la revendication 3, dans lequel le matériau de l'élément d'écrasement est un métal ayant une résistance spécifique.

5. Dispositif électronique selon la revendication 3, dans lequel au moins la partie de la cartouche écrasée par la chaleur est une cellule à couches minces.

6. Dispositif électronique selon la revendication 1, dans lequel le circuit d'écrasement comporte en outre un élément élastique (410) disposé entre une surface inférieure d'un substrat du circuit d'écrasement et la cartouche.

7. Dispositif électronique selon la revendication 6, dans lequel
le circuit d'écrasement comporte en outre un élément de libération d'émulsion (420) fixé à l'élément élastique, et
l'élément élastique et l'élément de libération d'émulsion sont configurés pour déplacer l'émulsion dans une direction de la micro-aiguille lorsqu'au moins la partie de la cartouche est écrasée par l'élément d'écrasement.

8. Dispositif électronique selon la revendication 1, comportant en outre :
un indicateur (214) indiquant une quantité d'émulsion à libérer.

9. Dispositif électronique selon la revendication 1, comportant en outre :
un bouton (212) configuré pour recevoir, d'un utilisateur, une quantité d'émulsion à libérer.

10. Dispositif électronique selon la revendication 1, dans lequel le processeur est configuré pour, lorsque la cartouche comporte une pluralité de blocs, chacun contenant une émulsion, générer le signal d'écrasement pour écraser un premier bloc (371, 391) de la pluralité de blocs.

11. Dispositif électronique selon la revendication 10, dans lequel
le circuit d'écrasement comporte une pluralité d'éléments d'écrasement, et
la pluralité d'éléments d'écrasement correspond respectivement à la pluralité de blocs.

12. Dispositif électronique selon la revendication 10, dans lequel
le vibreur à ultrasons comporte un ou plusieurs vibreurs à ultrasons, et
le processeur est configuré pour générer un premier signal de vibration pour commander un premier vibreur à ultrasons correspondant au premier bloc parmi le ou les vibreurs à ultrasons.
